# EUROPEAN PATENT SPECIFICATION

(11) **EP 2 254 516 B1**
(45) Date of publication and mention of the grant of the patent: **05.08.2015**
(21) Application number: 09703747.7
(22) Date of filing: 21.01.2009
(51) Int. Cl.: A61F 2/30, A61F 2/28

(54) **METHOD FOR FORMING AN INTEGRAL POROUS REGION IN CAST IMPLANT**
VERFAHREN ZUR BILDUNG EINER INTEGRALEN PORÖSEN REGION IN EINEM GIPSIMPLANTAT
PROCÉDÉ DE FORMATION D'UNE RÉGION POREUSE INTÉGRALE DANS UN IMPLANT COULÉ

(30) Priority: 21.01.2008 US 17123
(43) Date of publication of application: 01.12.2010
(73) Proprietor: Zimmer, Inc., Warsaw, IN 46580 (US)
(72) Inventor: RAUGUTH, Brad, North Webster IN 46555 (US); HUTCHISON, William, Warsaw IN 46582 (US)
(74) Representative: Mays, Julie
(86) International application number: PCT/US2009/031502
(87) International publication number: WO 2009/094354

(56) References cited:
- WO-A-93/07835
- WO-A-97/38649
- WO-A-2007/053022
- DE-C1- 4 106 971
- US-A- 5 535 810

## Description

### PRIORITY APPLICATION

This application claims priority to U.S. Patent Application No. 12/017,123 filed on January 21, 2008.

### TECHNOLOGY FIELD

This invention relates to casting prosthetic implants, and, more particularly, to casting prosthetic implants having integral porous regions formed therein.

### BACKGROUND

It is current practice to modify prosthetic devices, such as hip implants, to improve their attachment to bone tissue. For example, spraying a porous coating onto the implant is one type of modification. Usually spraying the porous coating is accomplished with one or more thermal spray processes. Another type of modification includes attachment of a porous body to the surface of the implant. The porous body may be attached to the surface of the implant by sintering or diffusion bonding the porous body to the implant. In each case, the goal is to provide a region on the implant which facilitates the growth of bone tissue into the implant. Growth of bone tissue within the porosity of the coating or porous body improves the mechanical integrity between natural tissue and the man-made implant. Thus, separation of the implant from adjacent tissue in vivo is less likely.

Unfortunately, there are drawbacks to each of the currently available modifications. For example, the porous bodies, either in the form of a coating or a separate porous component, are attached to the implant near the end of the manufacturing process. Each process, that is spraying or diffusion bonding, requires heating the implant to elevated temperatures. Problems caused by exposure to elevated temperatures include surface oxidation, stain formation, warping, and micro structural degradation of the implant. In other words, subsequent spray coating or diffusion bonding will result in an increase in the proportion of defective implants. In other words, there is a significant manufacturing cost associated with subsequent coating or bonding processes that exceeds the cost of the process itself.

US-A-5 535 810 discloses a method for forming an integral porous region in an orthopedic implant comprising: providing as implant pattern; attaching a porous framework into the pattern to a desired depth, forming a mold; heating the mold for removing the pattern from the mold but retaining the framework in the mold; filling the mold with a molten metal; cooling the mold; and removing the implant from the mold.

Thus, a method of forming a cast implant with a porous region where the implant is not exposed to elevated temperature subsequent to casting is needed.

### SUMMARY

Certain features, aspects and embodiments described herein are directed to methods for forming an integral porous region in an orthopedic implant. In one embodiment, the method comprises providing an orthopedic implant pattern, attaching a porous framework having a plurality of reticulated interstices into the orthopedic implant pattern to a desired depth, forming a mold by encasing the orthopedic implant pattern and the porous framework in a mold material, and removing the orthopedic implant pattern from the mold while retaining the porous framework in the mold. The mold, therefore, has an interior surface formed by the orthopedic implant pattern and the porous framework. Once the mold is prepared, it is heated and then filled with a molten metal. The molten metal enters at least a portion of the reticulated interstices of the porous framework. The mold, porous framework, and molten metal are then cooled, whereupon the orthopedic implant forms with an exterior surface that conforms to the interior surface of the mold and to the portion of the reticulated interstices filled with molten metal. The orthopedic implant is then removed from the mold. Finally, the porous framework is removed from the orthopedic implant to expose an integral porous region having a plurality of reticulated pores.

In another embodiment, a method for forming an integral porous region in an orthopedic implant comprises providing an orthopedic implant pattern, attaching a soluble porous framework having a plurality of reticulated interstices to the orthopedic implant pattern to a desired depth, and then dissolving the soluble porous framework with a solvent. A replica region is formed to the desired depth as the soluble porous framework dissolves. A mold is formed by encasing the orthopedic implant pattern in a mold material. The mold material penetrates into the replica region to form an integrated porous framework having a plurality of integrated reticulated interstices. The orthopedic implant pattern is then removed from the mold. The mold has an interior surface formed by the orthopedic implant pattern and the replica region. An integrated porous framework having a plurality of integrated reticulated interstices is formed from the replica region. Next the mold is heated and molten metal is poured therein such that the molten metal enters the integrated reticulated interstices. An orthopedic implant is formed as the mold and the molten metal cool. The mold is then removed to expose the orthopedic implant having a plurality of reticulated pores.

### BRIEF DESCRIPTION OF THE FIGURES

The accompanying drawings, which are incorporated in and constitute a part of this specification, illustrate embodiments and, together with a general description given above, and the detailed description given below, serve to explain certain features, aspects and embodiments of the technology described herein.
FIG. 1 depicts an elevation view of an orthopedic implant having an integral porous region according to an embodiment;
FIG. 2 depicts an elevation view of one embodiment of an orthopedic implant pattern;
FIG. 3A depicts an elevation view of the orthopedic implant pattern of FIG. 2 showing attachment of a porous framework to the orthopedic implant pattern;
FIGS. 3B and 3C depict enlarged cross-sectional views of an encircled area 3B of FIG. 3A illustrating exemplary embodiments of the porous framework;
FIG. 4 depicts a cross-sectional view of a mold formed around the orthopedic implant pattern with the porous framework attached thereto;
FIG. 4A depicts an enlarged view of an encircled area 4A of FIG. 4 illustrating penetration of the mold and the pattern into the porous framework;
FIG. 5 depicts a cross-sectional view of the mold with the porous framework retained therein with molten metal being poured into the mold;
FIG. 5A depicts an enlarged view of an encircled area 5A of FIG. 5 illustrating penetration of the molten metal into the porous framework;
FIG. 6 depicts an enlarged cross-sectional view of one embodiment of the integral porous region of an encircled area 6 of FIG. 1;
FIG. 7 depicts an elevation view of another embodiment of an orthopedic implant pattern showing attachment of a soluble porous framework to the pattern;
FIG. 8 depicts a portion of the pattern following dissolving the soluble porous framework of FIG. 7;
FIG. 9 depicts an enlarged cross-sectional view of an encircled area 9 of FIG. 8, illustrating one embodiment of a replica region;
FIG. 10 depicts a cross-sectional view of a mold formed around the orthopedic implant pattern with the replica region of FIG. 9;
FIG. 10A depicts an enlarged cross-sectional view of an encircled area 10A of the mold of FIG. 10;
FIG. 10B depicts an enlarged cross-sectional view of an encircled area 10A of FIG. 10 illustrating a penetration depth of the mold into the replica region;
FIG. 11 depicts a cross-sectional view of the mold of FIG. 10 with an integrated framework region with molten metal being poured therein; and
FIG. 11A depicts an enlarged cross-sectional view of an encircled area 11A of FIG. 11.

### DETAILED DESCRIPTION

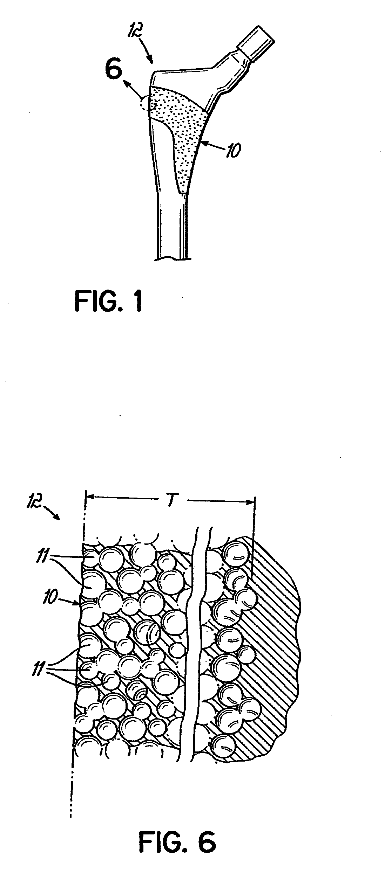

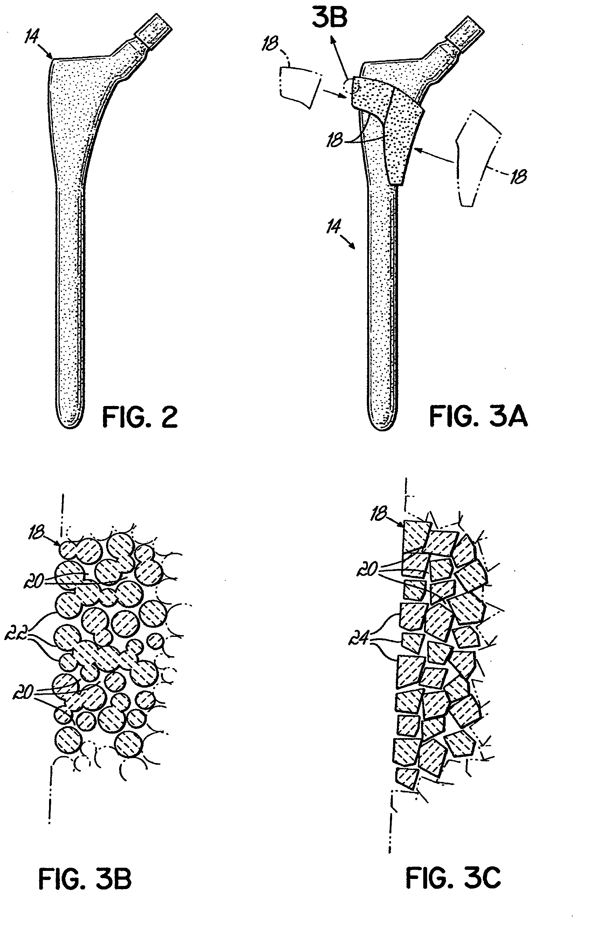

FIGS. 2 - 11 depict methods for casting an integral porous region 10 in an orthopedic implant 12, shown in FIG. 1, according to the principles disclosed herein. The integral porous region 10 comprises a plurality of reticulated pores 11 (also referred to as reticulated porosity 11 shown in FIG. 6) and is thus adapted to facilitate growth of living tissue therein, e.g., osseointegration in the case of bone, when the orthopedic implant 12 is surgically implanted into a living body. To this end, the integral porous region 10 is formed in the orthopedic implant 12 during casting such that the integral porous region 10 comprises the same metal as the orthopedic implant 12. Therefore, in an exemplary embodiment the orthopedic implant 12 is a monolithic metallic body having an integral porous metal region 10.

With reference specifically to FIG. 2, in one method an orthopedic implant pattern 14 is provided having the shape of the implant 12 (shown in FIG. 1) desired. In FIG. 2, the orthopedic implant pattern 14 is in the form of a hip implant. It will be appreciated that other orthopedic implants may be cast in accordance with this disclosure, e.g., knee, shoulder, spine, hip, elbow or other trauma products or implants. The orthopedic implant pattern 14 may comprise wax, plastic, other low melting or sacrificial materials or combinations thereof.

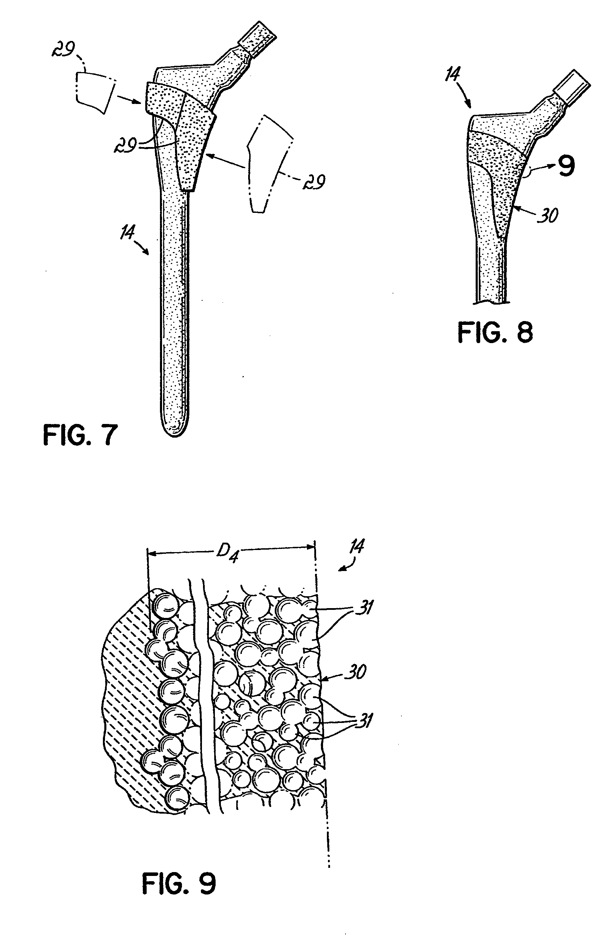

Next, as shown in FIGS. 3A- 3C, a porous framework 18 having a plurality of reticulated interstices 20, shown most clearly in FIGS. 3B and 3C, that extends throughout the porous framework 18, is attached to the pattern 14. With reference to FIG. 3A, the porous framework 18 may comprise one or more pieces. In one embodiment, the porous framework 18 is a porous ceramic framework. For example, the porous framework 18 may comprise zirconia, alumina, silica, or a combination of two or more of these materials. In another embodiment, the porous framework 18 comprises a soluble material, such as a wax, as will be described in detail later with reference to FIGS. 7-11A. FIGS. 3B and 3C illustrate embodiments of the porous framework 18. As shown, the porous framework 18 comprises the reticulated interstices 20, or void space, that extend throughout the porous framework 18. Furthermore, the reticulated interstices 20 are open to the atmosphere.

In an exemplary embodiment shown in FIG. 3B, the porous framework 18 comprises a plurality of beads 22 that are interconnected. The beads 22 may be arranged into layers or randomly packed together. The beads 22 are then attached together by methods known by those skilled in the art, such as one or more sintering processes. It will be appreciated that layering or packing the beads 22 together forms interstices 20 between adjacent beads 22, as shown. Furthermore, of the interstices 20 between adjacent beads 22 are interconnected so as to form a network of interstices 20, referred to as reticulated interstices 20. While not shown in the two dimensional figures of FIGS. 3B and 3C, the reticulated interstices 20 extend in three dimensions within the porous framework 18. It will be appreciated that variation in the size and shape of the adjacent beads 22 may change the size, shape, and interconnectivity of the reticulated interstices 20.

In the exemplary embodiment, shown in FIG. 3C, the porous framework 18 comprises a plurality of polyhedrons 24 that are interconnected. The polyhedrons 24 are arranged in contact with and attached to other polyhedrons 24. The polyhedrons 24 may be any one or a combination of regular or irregular polyhedrons that, when packed or placed into contact and attached to one another, form interstitial spaces that collectively form the reticulated interstices 20. As with the beads 22, previously discussed, variation in the size and shape of the polyhedrons 24 may change the size and shape of the reticulated interstices 20. It will be appreciated that many particle shapes and sizes may be placed in contact with and attached to one another to form the reticulated interstices 20. In one exemplary embodiment, the beads 22 or polyhedrons 24 may be attached or interconnected to one another by sintering the porous framework 18, as is known in the mi, prior to attaching it to the pattern 14. In one embodiment, the beads 22, the polyhedrons 24, or other particles, comprise a refractory material that is able to withstand contact with molten metal, such as ceramic materials and refractory metals.

As previously disclosed, not only can the form of the reticulated interstices 20 be modified, but the size and shape of the porous framework 18 may be varied. Generally, the size and shape of the integral porous region 10 may be made according to the recipient's physiological needs. For example, the size and shape of the porous framework 18 may be designed to provide an osseointegration pathway between a prosthetic hip implant stem and the bone surrounding the medullary canal.

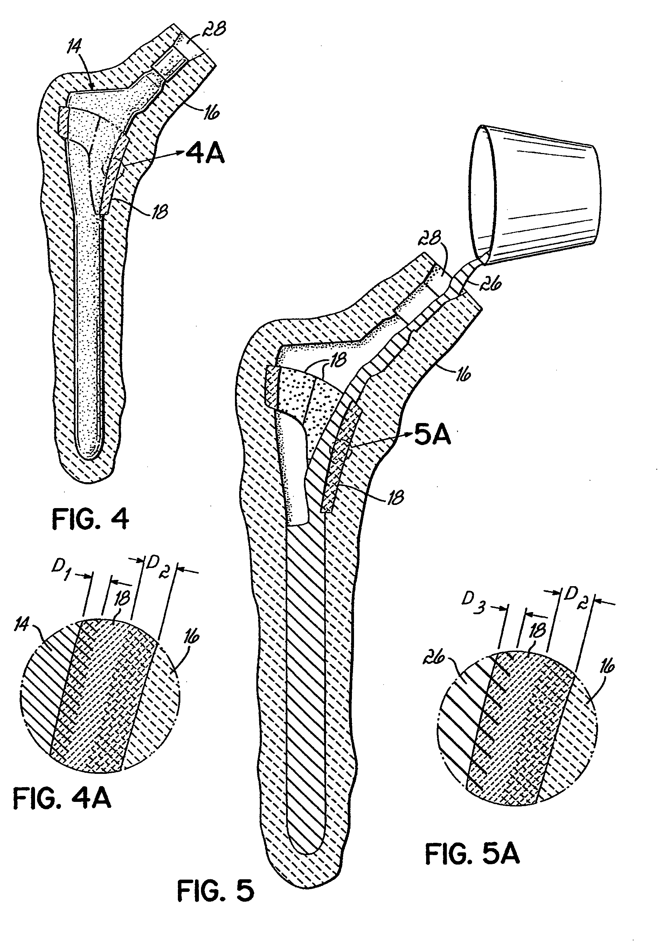

In one method, attaching the porous framework 18, as shown in FIG. 3A, may include pressing the porous framework 18 into the surface of the orthopedic implant pattern 14 to penetrate the beads 22 or polyhedrons 24, for example, into the pattern 14 whereby a portion of the pattern 14 enters the reticulated interstices 20 (to a penetration depth, D₁, as will be discussed below in reference to FIG. 4A). Attaching the porous framework 18 may include attaching multiple frameworks 18 to the pattern 14. For example, two frameworks 18 are attached to the pattern 14 as shown in FIG. 3A. In addition, the framework 18 may cover a small portion of the pattern 14 to the entire surface of the pattern 14 depending on the type of implant desired and the recipient's physiological needs, as previously mentioned. Moreover, the framework 18 may extend through the pattern 14 such that portions of the framework 18 project from one or more surfaces of the pattern 14.

Now with reference to FIG. 4, the mold 16 is formed around the pattern 14 and the porous framework 18. For example, the mold 16 may be formed by dipping the pattern 14 and the porous framework 18 into a mold material comprising ceramic particles (e.g., a slurry), as is known in the art. Once the slurry dries, the pattern 14 and the porous framework 18 may be encased in the mold 16. Alternatively, the mold 16 may be formed by spraying a ceramic slurry onto the pattern 14 and the porous framework 18 and allowing the slurry to dry. In addition to covering the pattern 14, some of the ceramic particles enter the reticulated interstices 20 of the porous framework 18 not filled by the pattern 14.

With reference to FIG. 4A, the penetration depth, D₁, may represent the penetration of the pattern 14 into the porous framework 18, specifically into the reticulated interstices 20 (not shown). In one exemplary embodiment, and with reference to FIG. 4A, the porous framework 18 is pressed into the pattern 14 to a penetration depth, D₁, of about 2 mm to about 4 mm. That is, the pattern 14 penetrates about 2 mm to about 4 mm into the reticulated interstices 20. As will be described later, D₁ may determine the thickness of the integral porous region 10 in the implant 12.

According to one embodiment, while forming the mold 16 as illustrated in FIGS. 4 and 4A, ceramic particles may penetrate to a depth, D₂, into the porous framework 18. It will be appreciated that the mold material may penetrate into the reticulated interstices 20 proximate the pattern 14, or, as shown or in FIG. 4A, the mold material may fill to a depth D₂ that is something less than the available portion of the porous framework 18, specifically the reticulated interstices 20. Therefore, while reference may be made herein that the mold material penetrates the remaining portion of the reticulated interstices 20 not filled by the pattern 14, the mold material need not fill all of the remaining reticulated interstices 20. For example, as shown in FIG. 4A, a portion of the porous framework 18 between the pattern material and the mold material may remain unfilled.

As will be appreciated by those skilled in the art, other mold materials may be used to encase the pattern 14 and the porous framework 18 to form the mold 16. For example, investment casting slurries may comprise zirconia, silica, alumina, a combination of these materials or another similar material that hardens or sets may be used to form the mold 16. To that end, materials that conform to the pattern 14, that can penetrate into the reticulated interstices 20, and that can withstand contact with molten metal may be used.

Once the mold 16 is formed, the pattern 14 is removed. In one exemplary embodiment, removing the pattern 14 from the mold 16 may include, for example, heating the mold 16 and the pattern 14 to a temperature to melt the pattern 14. The melted pattern may then be poured from the mold 16 and the reticulated interstices 20.

With reference now to FIG. 5, while the pattern 14 is removed from the mold 16 and the reticulated interstices 20, the porous framework 18 is retained by the mold 16. In other words, as shown in FIG. 5, the mold 16 has an interior surface formed by the pattern 14 and a portion of the porous framework 18 not filled by the mold 16. In one embodiment, once the slurry dries and the pattern 14 is removed therefrom, the mold 16 may be fired or subject to an additional heat treatment.

With continued reference to FIG. 5, once the pattern 14 (shown in FIG. 4) is removed from the mold 16 and the reticulated interstices 20, a molten metal 26 is poured into the mold 16. In one exemplary embodiment, the molten metal 26 comprises a cobalt-chromium alloy. However, the molten metal 26 may be other metals, for example, titanium, a titanium alloy, or a zirconium alloy, or other metal or alloy that is biocompatible. In one embodiment, the mold 16 and the porous framework 18 may be preheated to a temperature sufficient to prevent thermal shock of the mold 16 when initially contacted by the molten metal 26. As shown in FIG. 5, the molten metal 26 may flow into the mold 16 through aperture 28. However, it will be appreciated that a gating (not shown) may attach the mold 16 to a runner system (not shown) to deliver the molten metal to multiple molds 16. It will also be appreciated that multiple apertures 28 may be required to cast more complex implants.

With reference to FIG. 5A, the molten metal 26 fills at least a portion of the reticulated interstices 20 (not shown) in the porous framework 18 to a depth, D₃. The depth D₃ may be proportional to the penetration depth, D₁ of the pattern 14, as shown in FIG. 4A. One skilled in the art will appreciate that the molten metal 26 may penetrate into the porous framework 18 proximate the mold material in the reticulated interstices 20. In one exemplary embodiment, the molten metal 26 fills the portion of the reticulated interstices 20 that is not filled by the mold 16. That is, the molten metal 26 may flow into contact with the mold 16 such that the two penetration depths D₃ and D₂ abut. However, the molten metal 26 need not fill all of the reticulated interstices 20 not filled by the mold 16, as shown in FIG. 5A. The orthopedic implant 12 shown in FIG. 1 is formed following cooling of the mold 16 and the porous framework 18.

The orthopedic implant 12 is then removed from the mold 16 to expose the exterior surface of the orthopedic implant 12. In one embodiment, mechanical impact, such as that caused by a hammer, is used to fracture or break the mold 16. Thus, the orthopedic implant 12 is freed from the mold 16 by breaking the mold 16 into pieces. Similarly, any residual porous framework 18 may be removed from the orthopedic implant 12 by mechanical impact. In one embodiment, a grit blasting or a loose abrasive process is used to remove the porous framework 18 from the orthopedic implant 12. In another embodiment, removing the porous framework 18 includes chemically dissolving the porous framework 18, such as with an acid or other solvent that preferentially dissolves the porous framework 18 but does not appreciably degrade the implant 12.

The orthopedic implant 12 of FIG. 1 has an exterior surface that may conform to the interior surface of the mold 16 and a portion of the reticulated interstices 20 in the porous framework 18. In particular, the integral porous region 10 may be defined by the portion of the reticulated interstices 20 that is filled by the molten metal 26. It will be appreciated that filling a portion of the reticulated interstices 20 will form the reticulated porosity 11, unlike prior art processes. In one embodiment, the integral porous region 10 is formed substantially flush with the exterior surface of the orthopedic implant 12, as shown in FIG. 1. In another embodiment, the penetration depth, D₁, of the pattern 14 within the reticulated interstices 20 (shown in FIG. 4A) determines thickness, T, of the integral porous region 10 illustrated in FIG. 6, which is an enlarged portion of the integral porous region 10 taken from FIG. 1. For example, if the porous framework 18 is pressed into the pattern 14 to a depth, D₁, as previously described, of about 2 mm to about 4 mm, the thickness T may also be from about 2 mm to about 4 mm, respectively. Generally, T will be proportional to D₁ such that the integral porous region 10 may be formed to any desired T to facilitate osseointegration. It will be appreciated that even though a boundary between the porous framework 18 and the mold 16 as well as the boundary between the pattern 14 and the porous framework 18, as depicted in FIG. 4A and other boundaries in other figures, described herein, are indicated by a line, the boundary may not be linear and are shown as such to simplify description of the penetration of the pattern 14 and the mold 16 into the porous framework 18.

In addition to varying T to facilitate, for example, osseointegration, the location of the exterior surface of the integral porous region 10 relative to the exterior surface of the implant 12 may be determined by both the penetration depths D₁ and D₂ relative to the exterior surface of the pattern 14. In one exemplary embodiment, the exterior surface of the implant 12 is formed flush with the exterior surface of the integral porous region 10, as shown in FIG. 1, which is unlike prior art coatings and diffusion bonding of porous bodies. On the other hand, by way of example and not limitation by varying the depths, D₁, D₂, and D₃, the integral porous region 10 may be recessed or project from the exterior surface of the implant 12. Thus, the exterior surface of the integral porous region 10 may not be flush with the exterior surface of the implant 12.

FIG. 6 illustrates one exemplary embodiment of the integral porous region 10 in the implant 12. Thus, in one embodiment, the integral porous region 10, for example of FIG. 6, is defined by the reticulated interstices 20 of FIG. 3B. The integral porous region 10 comprises the reticulated porosity 11 that is open to the atmosphere and may extend in three dimensions from the exterior surface of the integral porous region 10 into the implant 12. Consequently, unlike the prior art processes, biological fluids and tissue may flow in one or more directions into, through, and out of the integral porous region 10 via the reticulated porosity 11.

In another embodiment, as previously mentioned and with reference to FIGS. 7-11, the porous framework 18 comprises a soluble material, hereinafter referred to as a soluble porous framework 29. The soluble material dissolves in a solvent. The solvent may be water, an aqueous solvent, such as a mild acid in an aqueous solution, or one of many non-aqueous solvents that will not appreciably dissolve the pattern 14. The soluble porous framework 29 has a plurality of reticulated interstices 20 (not shown) similar to the porous framework 18. Accordingly, as shown in FIG. 7, the soluble porous framework 29 is attached to the pattern 14, as before. Thus, the pattern 14 enters a portion of the reticulated interstices 20 (not shown) of the soluble porous framework 29. However, once the soluble porous framework 29 is positioned, the solvent is used to remove the soluble porous framework 29 from the pattern 14. A replica region 30 is formed in the pattern 14 as shown in FIG. 8. With reference to FIG. 9, the replica region 30 has a depth of D₄ and open porosity 31 following removal of the soluble porous framework 29 therefrom.

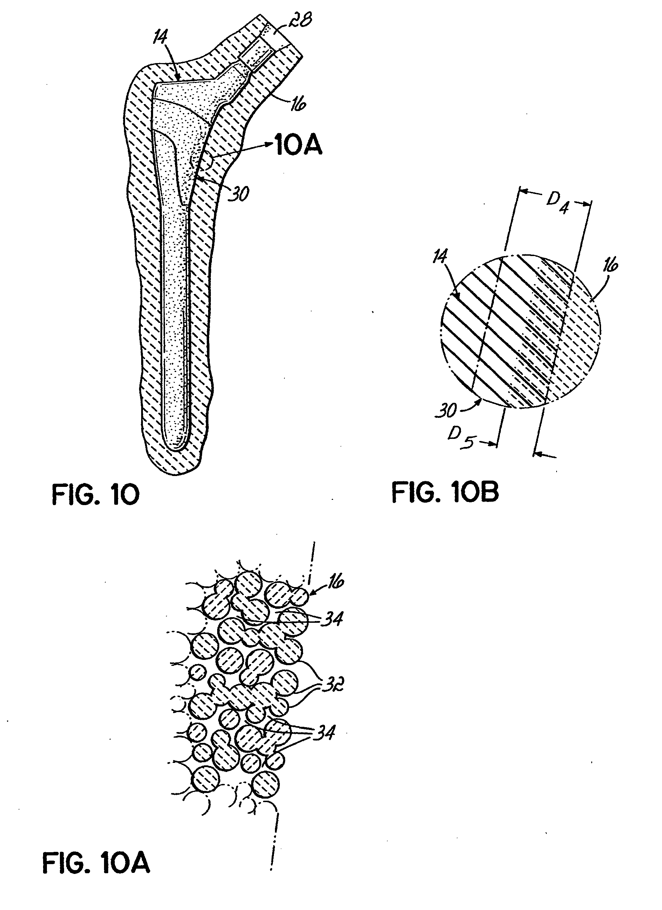

Therefore, once the soluble porous framework 29 is dissolved and removed, the mold 16 may be formed in a similar manner as before, as illustrated in FIG. 10. However, for example, if a mold material comprising a slurry of ceramic particles is used to form the mold 16, the slurry penetrates into the replica region 30. Furthermore, as illustrated in FIG. 10B, the mold material may penetrate into the replica region 30 of the pattern 14 to a depth D₅. The mold 16 may be dried or allowed to set, then the pattern 14 may be removed, for example, by heating the mold 16 and the pattern 14 to melt the pattern 14 and then pouring the melted pattern from the mold 16. An integrated porous framework 32, as shown in FIG. 10A, is formed by the open porosity 31 (shown in FIG. 9) in the mold 16. The integrated porous framework 32 of the mold 16 thus has a plurality of integral reticulated interstices 34.

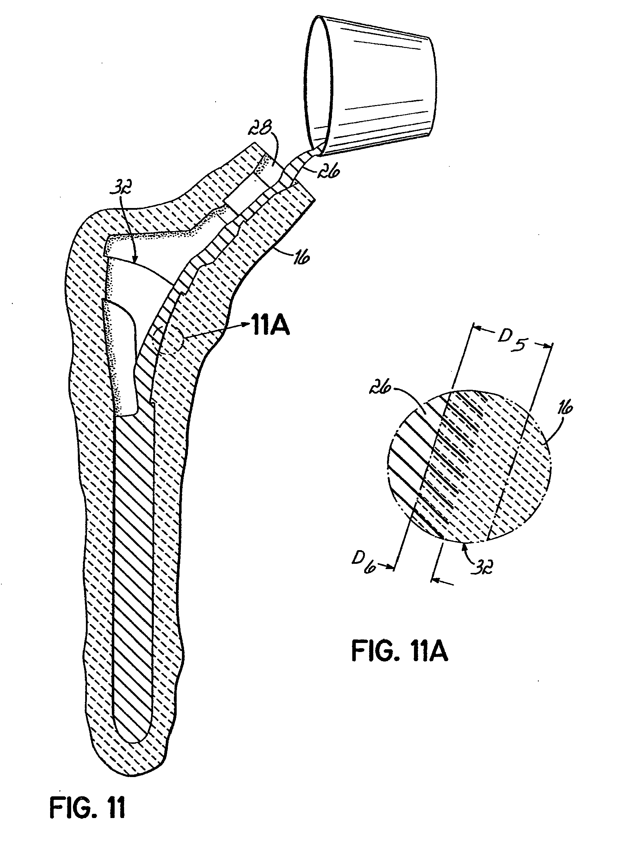

With reference now to FIG. 11, once the mold 16 is prepared to receive the molten metal 26, the molten metal 26 may be poured through an aperture 28 into the mold 16. As the molten metal 26 fills the mold 16, some of the molten metal 26 enters the integrated porous framework 32. In particular, the molten metal 26 penetrates into the integral reticulated interstices 34 to a depth D₆, as shown in FIG. 11A which may be proportional to the depth D₅. While D₆ may be less than D₅, in one exemplary embodiment, they are substantially the same. That is, the molten metal 26 fills substantially all of the integral reticulated interstices 34 shown in FIG. 10A.

After cooling, the implant 12 is formed having the integral porous region 10 as shown in FIG. 1. Thus, when the mold 16 is removed, the exterior surface of the integral porous region 10 may be substantially flush with the exterior surface of the implant 12. It will be appreciated that the exterior surface of the integral porous region 10 may be designed to project or be recessed relative to the exterior surface of the implant 12 by altering the penetration depths of D₄, D₅, and D₆. Generally, as the penetration depth D₄ of the replica region 30 increases, both the penetration depth D₅ of the mold material into the replica region 30 and the penetration depth D₆ of the molten metal 26 into the integrated porous framework 32 also increase. Therefore, modification of the soluble porous framework 29 will allow the depths D₄, D₅ and D₆ to vary, ultimately providing a means to tailor the implant 12 to the patients' needs.

While the present invention has been illustrated by the description of one or more embodiments thereof, and while the embodiments have been described in considerable detail, they are not intended to restrict or in any way limit the scope of the appended claims to such detail. Additional advantages and modifications will readily appear to those skilled in the art. The invention is therefore not limited to the specific details, representative apparatus and method and illustrative examples shown and described. Accordingly, departures may be made from such details without departing from the scope of the invention as defined by the appended claims.

## Claims

1. A method for forming an integral porous region (10) in an orthopedic implant (12) comprising:
providing an orthopedic implant pattern (14);
attaching a porous framework (18) having a plurality of reticulated interstices (20) into the orthopedic implant pattern (14) to a desired depth;
forming a mold (16) by encasing the orthopedic implant pattern (14) and the porous framework (18) in a mold material (16);
removing the orthopedic implant pattern (14) from the mold (16) and retaining the porous framework (18) in the mold (16), whereby the mold (16) has an interior surface formed by the orthopedic implant pattern (14) and the porous framework (18);
heating the mold (16) with the porous framework (18) retained therein;
filling the mold (16) with a molten metal (26) such that the molten metal (26) enters at least a portion of the reticulated interstices (20);
cooling the mold (16), the porous framework (18), and the molten metal (26) to form an orthopedic implant (12) with an exterior surface conforming to a mold (16) interior surface and to the portion of the reticulated interstices (20);
removing the orthopedic implant (12) from the mold (16) to expose the exterior surface of the orthopedic implant (12); and
removing the porous framework (18) from the orthopedic implant (12) to expose an integral porous region (10) having a plurality of reticulated pores (11).

2. The method of claim 1 wherein the porous framework (18) comprises a plurality of interconnected beads (22), and the reticulated interstices (20) are formed by the interconnected beads (22), and wherein the attaching includes pressing at least a portion of the plurality of interconnected beads (22) into the orthopedic implant pattern (14) to the desired depth whereby a portion of the orthopedic implant pattern (14) penetrates into at least a portion of the reticulated interstices (20).

3. The method of claim 1 wherein the porous framework (18) comprises a plurality of interconnected polyhedrons (24), and the reticulated interstices (20) are formed by the interconnected polyhedrons (24), and wherein attaching includes pressing at least a portion of the plurality of interconnected polyhedrons (24) into the orthopedic implant pattern (14) to the desired depth whereby a portion of the orthopedic implant pattern (14) penetrates into at least a portion of the reticulated interstices (20).

4. The method of claim 1 wherein following removing the mold (16), the integral porous region (10) is substantially flush with the exterior surface of the orthopedic implant (12).

5. The method of claim 1 wherein the orthopedic implant pattern (14) comprises a sacrificial material, and wherein removing the orthopedic implant pattern (14) from the mold (16) includes heating the mold (16) to a temperature above a melting temperature of the sacrificial material to melt the orthopedic implant pattern (14) and pouring the melted sacrificial material from the mold (16).

6. The method of claim 5 wherein the sacrificial material comprises a wax or a plastic material.

7. The method of claim 1 wherein forming the mold (16) includes dipping the orthopedic implant pattern (14) and the porous framework (18) in a slurry comprising ceramic particles.

8. The method of claim 1 wherein removing the orthopedic implant (12) from the mold (16) includes mechanically impacting the mold (16).

9. The method of claim 1 wherein removing the porous framework (18) from the orthopedic implant (12) includes mechanically impacting the porous framework (18).

10. The method of claim 1 wherein removing the porous framework (18) from the orthopedic implant (12) includes chemically dissolving the porous framework (18).

11. The method of claim 1 wherein the orthopedic implant pattern (14) comprises a wax material and the porous framework (18) comprises a ceramic material; and
wherein:
attaching the porous ceramic framework (18) to the orthopedic implant pattern (14) includes inserting a first portion of the porous ceramic framework (18) into the orthopedic implant pattern (14) whereby a second portion of the porous ceramic framework (18) protrudes from an exterior surface of the orthopedic implant pattern (14);
forming the mold (16) includes dipping the orthopedic implant pattern (14) and the porous ceramic framework (18) in a slurry comprising ceramic particles whereby the slurry fills at least a portion of the reticulated interstices (20) in the second portion of the porous ceramic framework (18); and drying the slurry;
removing the orthopedic implant pattern (14) from the mold (16) and retaining the porous ceramic framework (18) in the mold (16) includes melting the wax and pouring the melted wax out of the mold (16), whereby the mold (16) has an interior surface formed by the orthopedic implant pattern (14) and the first portion of the porous ceramic framework (18);
and
filling the mold (16) includes pouring a molten metal (26) into the mold (16) such that the molten metal (26) enters the reticulated interstices (20) in the first portion as the molten metal (26) fills the mold (16).

12. A method for forming an integral porous region (10) in an orthopedic implant (12) comprising:
providing an orthopedic implant pattern (14);
attaching a soluble porous framework (29) having a plurality of reticulated interstices (20) into the orthopedic implant pattern (14) for a desired depth;
dissolving the soluble porous framework (29) with a solvent, whereby a replica region (30) is formed to the desired depth in the orthopedic implant pattern (14);
forming a mold (16) by encasing the orthopedic implant pattern (14) in a mold material (16), whereby the mold material (16) penetrates into the replica region (30);
removing the orthopedic implant pattern (14) from the mold (16), whereby the mold (16) has an interior surface formed by the orthopedic implant pattern (14) and the replica region (30) and whereby an integrated porous framework (29) having a plurality of integrated reticulated interstices (34) is formed;
heating the mold (16);
filling the mold (16) with a molten metal (26) such that the molten metal (26) enters the integrated reticulated interstices (34);
cooling the mold (16) and the molten metal (26) to form an orthopedic implant (12) with an exterior surface conforming to a mold (16) interior surface and the integrated reticulated interstices (34);
removing the orthopedic implant (12) from the mold (16) to expose the exterior surface of the orthopedic implant (12) having an integral porous region (10) having a plurality of reticulated pores (11).

## Patentansprüche

1. Verfahren zum Ausbilden eines integrierten porösen Bereichs (10) in einem orthopädischen Implantat (12), das Folgendes umfasst:
Bereitstellen einer Schablone (14) für ein orthopädisches Implantat;
Befestigen eines porösen Rahmens (18) mit mehreren netzförmig ausgebildeten Zwischenräumen (20) in der Schablone (14) für ein orthopädisches Implantat bis auf eine erwünschte Tiefe;
Ausbilden einer Form (16) durch Einschließen der Schablone (14) für ein orthopädisches Implantat und des porösen Rahmens (18) in ein Formmaterial (16);
Entfernen der Schablone (14) für ein orthopädisches Implantat aus der Form (16) und Behalten des porösen Rahmens (18) in der Form (16), wodurch die Form (16) eine Innenfläche aufweist, die von der Schablone (14) für ein orthopädisches Implantat und dem porösen Rahmen (18) ausgebildet wurde;
Erwärmen der Form (16) mit dem darin enthaltenen porösen Rahmen (18);
Füllen der Form (16) mit einer Metallschmelze (26), so dass die Metallschmelze (26) in wenigstens einen Teil der netzförmig ausgebildeten Zwischenräume (20) eintritt;
Abkühlen der Form (16), des porösen Rahmens (18) und der Metallschmelze (26), um ein orthopädisches Implantat (12) mit einer Außenfläche zu bilden, die einer Innenfläche einer Form (16) und dem Teil der netzförmig ausgebildeten Zwischenräume (20) entspricht;
Entfernen des orthopädischen Implantats (12) aus der Form (16), um die Außenfläche des orthopädischen Implantats (12) freizulegen; und
Entfernen des porösen Rahmens (18) von dem orthopädischen Implantat (12), um einen integrierten porösen Bereich (10) mit mehreren netzförmig ausgebildeten Poren (11) freizulegen.

2. Verfahren nach Anspruch 1, wobei der poröse Rahmen (18) mehrere miteinander verbundene Kügelchen (22) aufweist und die netzförmig ausgebildeten Zwischenräume (20) durch die miteinander verbundene Kügelchen (22) gebildet werden und wobei das Anbringen beinhaltet, dass wenigstens ein Teil der mehreren miteinander verbundenen Kügelchen (22) bis auf die erwünschte Tiefe in die Schablone (14) für ein orthopädisches Implantat gepresst wird, wobei ein Teil der Schablone (14) für ein orthopädisches Implantat in wenigstens einen Teil der netzförmig ausgebildeten Zwischenräume (20) eindringt.

3. Verfahren nach Anspruch 1, wobei der poröse Rahmen (18) mehrere miteinander verbundene Polyeder (24) aufweist und die netzförmig ausgebildeten Zwischenräume (20) von den miteinander verbundenen Polyedern (24) gebildet werden und wobei das Anbringen beinhaltet, dass wenigstens ein Teil der mehreren miteinander verbundenen Polyeder (24) bis auf die erwünschte Tiefe in die Schablone (14) für ein orthopädisches Implantat gepresst wird, wobei ein Teil der Schablone (14) für ein orthopädisches Implantat in wenigstens einen Teil der netzförmig ausgebildeten Zwischenräume (20) eindringt.

4. Verfahren nach Anspruch 1, wobei nach dem Entfernen der Form (16) der integrierte poröse Bereich (10) im Wesentlichen mit der Außenfläche des orthopädischen Implantats (12) bündig ist.

5. Verfahren nach Anspruch 1, wobei die Schablone (14) für ein orthopädisches Implantat ein Opfermaterial enthält und wobei das Entfernen der Schablone (14) für ein orthopädisches Implantat aus der Form (16) das Erwärmen der Form (16) auf eine Temperatur über dem Schmelzpunkt des Opfermaterials umfasst, um die Schablone (14) für ein orthopädisches Implantat zu schmelzen und das geschmolzene Opfermaterial aus der Form (16) auszugießen.

6. Verfahren nach Anspruch 5, wobei das Opfermaterial ein Wachs oder ein Kunststoffmaterial umfasst.

7. Verfahren nach Anspruch 1, wobei das Ausbilden der Form (16) das Eintauchen der Schablone (14) für ein orthopädisches Implantat und des porösen Rahmens (18) in eine Keramikpartikel enthaltende Aufschlämmung umfasst.

8. Verfahren nach Anspruch 1, wobei das Entfernen des orthopädischen Implantats (12) aus der Form (16) ein mechanisches Einwirken auf die Form (16) umfasst.

9. Verfahren nach Anspruch 1, wobei das Entfernen des porösen Rahmens (18) aus dem orthopädischen Implantat (12) ein mechanisches Einwirken auf den porösen Rahmen (18) umfasst.

10. Verfahren nach Anspruch 1, wobei das Entfernen des porösen Rahmens (18) aus dem orthopädischen Implantat (12) ein chemisches Auflösen des porösen Rahmens (18) umfasst.

11. Verfahren nach Anspruch 1, wobei die Schablone (14) für ein orthopädisches Implantat ein Wachsmaterial aufweist und der poröse Rahmen (18) ein Keramikmaterial aufweist; und
wobei
das Anbringen des porösen keramischen Rahmens (18) an die Schablone (14) für ein orthopädisches Implantat das Einsetzen eines ersten Abschnitts des porösen keramischen Rahmens (18) in die Schablone (14) für ein orthopädisches Implantat umfasst, wobei ein zweiter Abschnitt des porösen keramischen Rahmens (18) aus einer Außenfläche der Schablone (14) für ein orthopädisches Implantat herausragt;
das Ausbilden der Form (16) ein Eintauchen der Schablone (14) für ein orthopädisches Implantat und des porösen keramischen Rahmens (18) in eine Keramikpartikel enthaltende Aufschlämmung umfasst, wodurch die Aufschlämmung wenigstens einen Teil der netzförmig ausgebildeten Zwischenräume (20) im zweiten Abschnitt des porösen keramischen Rahmens (18) ausfüllt; und Trocknen der Aufschlämmung;
das Entfernen der Schablone (14) für ein orthopädisches Implantat aus der Form (16) und das Behalten des porösen keramischen Rahmens (18) in der Form (16) ein Schmelzen des Wachses und Ausgießen des geschmolzenen Wachses aus der Form (16) umfasst, wodurch die Form (16) eine Innenfläche aufweist, die von der Schablone (14) für ein orthopädisches Implantat und dem ersten Abschnitt des porösen keramischen Rahmens (18) ausgebildet wurde;
und
das Füllen der Form (16) ein Gießen der Metallschmelze (26) in die Form (16) beinhaltet, so dass die Metallschmelze (26) in die netzförmig ausgebildeten Zwischenräume (20) im ersten Abschnitt eintritt, während die Metallschmelze (26) die Form (16) ausfüllt.

12. Verfahren zum Ausbilden eines integrierten porösen Bereichs (10) in einem orthopädischen Implantat (12), das Folgendes umfasst:
Bereitstellen einer Schablone (14) für ein orthopädisches Implantat;
Befestigen eines löslichen porösen Rahmens (29) mit mehreren netzförmig ausgebildeten Zwischenräumen (20) in der Schablone (14) für ein orthopädisches Implantat für eine erwünschte Tiefe;
Auflösen des löslichen porösen Rahmens (29) mit einem Lösungsmittel, wodurch ein Replikbereich (30) bis auf die erwünschte Tiefe in der Schablone (14) für ein orthopädisches Implantat ausgebildet wird;
Ausbilden einer Form (16) durch Einschließen der Schablone (14) für ein orthopädisches Implantat in ein Formmaterial (16), wobei das Formmaterial (16) in den Replikbereich (30) eindringt;
Entfernen der Schablone (14) für ein orthopädisches Implantat aus der Form (16), wodurch die Form (16) eine Innenfläche aufweist, die von der Schablone (14) für ein orthopädisches Implantat und dem Replikbereich (30) ausgebildet wurde, und wodurch ein integrierter poröser Rahmen (29) mit mehreren integrierten, netzförmig ausgebildeten Zwischenräumen (34) ausgebildet wird;
Erwärmen der Form (16);
Füllen der Form (16) mit einer Metallschmelze (26), so dass die Metallschmelze (26) in die integrierten, netzförmig ausgebildeten Zwischenräume (34) eintritt;
Abkühlen der Form (16) und der Metallschmelze (26), um eine orthopädisches Implantat (12) mit einer Außenfläche zu bilden, die einer Innenfläche einer Form (16) und den integrierten, netzförmig ausgebildeten Zwischenräumen (34) entspricht;
Entfernen des orthopädischen Implantats (12) aus der Form (16), um die Außenfläche des orthopädischen Implantats (12), die einen integrierten porösen Bereich (10) mit mehreren netzförmig ausgebildeten Poren (11) aufweist, freizulegen.

## Revendications

1. Procédé destiné à former une région (10) poreuse intégrale dans un implant (12) orthopédique consistant à :
fournir un motif (14) d'implant orthopédique ;
fixer une structure (18) poreuse présentant une pluralité d'interstices (20) réticulés dans le motif (14) d'implant orthopédique à une profondeur souhaitée ;
former un moule (16) en enfermant le motif (14) d'implant orthopédique et la structure (18) poreuse dans une matière (16) de moulage ;
enlever le motif (14) d'implant orthopédique du moule (16) et retenir la structure (18) poreuse dans le moule (16), le moule (16) ayant une surface intérieure formée par le motif (14) d'implant orthopédique et la structure (18) poreuse ;
chauffer le moule (16) avec la structure (18) poreuse retenue à l'intérieur de ce dernier ;
remplir le moule (16) d'un métal (26) en fusion de sorte que le métal (26) en fusion entre au moins dans une partie des interstices (20) réticulés ;
refroidir le moule (16), la structure (18) poreuse, et le métal (26) en fusion pour former un implant (12) orthopédique présentant une surface extérieure épousant les contours d'une surface intérieure du moule (16) et de la partie des interstices (20) réticulés ;
enlever l'implant (12) orthopédique du moule (16) pour exposer la surface extérieure de l'implant (12) orthopédique ; et
enlever la structure (18) poreuse de l'implant (12) orthopédique pour exposer une région (10) poreuse intégrale présentant une pluralité de pores (11) réticulés.

2. Procédé selon la revendication 1 dans lequel la structure (18) poreuse comporte une pluralité de perles (22) reliées entre elles, et les interstices (20) réticulés sont formés par les perles (22) reliées entre elles, et dans lequel la fixation consiste à comprimer au moins une partie de la pluralité des perles (22) reliées entre elles dans le motif (14) d'implant orthopédique jusqu'à la profondeur souhaitée, une partie du motif (14) d'implant orthopédique pénétrant dans au moins une partie des interstices (20) réticulés.

3. Procédé selon la revendication 1 dans lequel la structure (18) poreuse comporte une pluralité de polyèdres (24) reliés entre eux, et les interstices (20) réticulés sont formés par les polyèdres (24) reliés entre eux, et dans lequel la fixation consiste à comprimer au moins une partie de la pluralité des polyèdres (24) reliés entre eux dans le motif (14) d'implant orthopédique jusqu'à la profondeur souhaitée, une partie du motif (14) d'implant orthopédique pénétrant dans au moins une partie des interstices (20) réticulés.

4. Procédé selon la revendication 1 dans lequel après avoir en enlevé le moule (16), la région (10) poreuse intégrale est sensiblement en affleurement avec la surface extérieure de l'implant (12) orthopédique.

5. Procédé selon la revendication 1 dans lequel le motif (14) d'implant orthopédique comporte une matière sacrificielle, et dans lequel l'enlèvement du motif (14) d'implant orthopédique du moule (16) consiste à chauffer le moule (16) à une température au-dessus d'une température de fusion de la matière sacrificielle pour faire fondre le motif (14) d'implant orthopédique et verser la matière sacrificielle fondue hors du moule (16).

6. Procédé selon la revendication 5 dans lequel la matière sacrificielle comporte une cire ou une matière plastique.

7. Procédé selon la revendication 1 dans lequel le formage du moule (16) consiste à tremper le motif (14) d'implant orthopédique et la structure (18) poreuse dans une boue comportant des particules de céramique.

8. Procédé selon la revendication 1 dans lequel l'enlèvement de l'implant (12) orthopédique du moule (16) consiste à impacter mécaniquement le moule (16).

9. Procédé selon la revendication 1 dans lequel l'enlèvement de la structure (18) poreuse de l'implant (12) orthopédique consiste à impacter mécaniquement la structure (18) poreuse.

10. Procédé selon la revendication 1 dans lequel l'enlèvement de la structure (18) poreuse de l'implant (12) orthopédique consiste à dissoudre chimiquement la structure (18) poreuse.

11. Procédé selon la revendication 1 dans lequel le motif (14) d'implant orthopédique comporte une matière en cire et la structure (18) poreuse comporte une matière en céramique ; et
où
la fixation de la structure (18) poreuse en céramique sur le motif (14) d'implant consiste à insérer une première partie de la structure (18) poreuse céramique dans le motif (14) d'implant orthopédique, une seconde partie de la structure (18) poreuse en céramique faisant saillie depuis une surface extérieure du motif (14) d'implant orthopédique ;
le formage du moule (16) consiste à tremper le motif (14) d'implant orthopédique et la structure (18) poreuse en céramique dans une boue comportant des particules de céramique, la boue remplissant au moins une partie des interstices (20) réticulés dans la seconde partie de la structure (18) poreuse en céramique ; et à sécher la boue ;
l'enlèvement du motif (14) d'implant orthopédique du moule (16) et la retenue de la structure (18) poreuse en céramique dans le moule (16) consiste à faire fondre la cire et à verser la cire fondue hors du moule (16), le moule (16) présentant une surface intérieure formée par le motif (14) d'implant orthopédique et la première partie de la structure (18) poreuse en céramique ;
et
le remplissage du moule (16) consiste à verser un métal (26) en fusion dans le moule (16) de sorte que le métal (26) en fusion entre dans les interstices (20) réticulés dans la première partie au fur et à mesure que le métal (26) en fusion remplit le moule (16).

12. Procédé destiné à former une région (10) poreuse intégrale dans un implant (12) orthopédique consistant à :
fournir un motif (14) d'implant orthopédique ;
fixer une structure (29) poreuse soluble présentant une pluralité d'interstices (20) réticulés dans le motif (14) d'implant orthopédique à une profondeur souhaitée ;
dissoudre la structure (29) poreuse soluble avec un solvant, une région (30) de duplication étant formée à la profondeur souhaitée dans le motif (14) d'implant orthopédique ;
former un moule (16) en enfermant le motif (14) d'implant orthopédique dans une matière (16) de moulage, la matière (16) de moulage pénétrant dans la région (30) de duplication ;
enlever le motif (14) d'implant orthopédique du moule (16), le moule (16) présentant une surface intérieure formée par le motif (14) d'implant orthopédique et la région (30) de duplication et une structure (29) intégrée poreuse présentant une pluralité d'interstices (34) réticulés intégrés s'y étant formée ;
chauffer le moule (16) ;
remplir le moule (16) d'un métal (26) en fusion de sorte que le métal (26) en fusion entre dans les interstices (34) réticulés intégrés ;
refroidir le moule (16) et le métal (26) en fusion pour former un implant (12) orthopédique présentant une surface extérieure épousant les contours d'une surface intérieure du moule (16) et des interstices (34) réticulés intégrés ;
enlever l'implant (12) orthopédique du moule (16) pour exposer la surface extérieure de l'implant (12) orthopédique munie d'une région (10) poreuse intégrale présentant une pluralité de pores (11) réticulés.
